# EUROPEAN PATENT SPECIFICATION

(11) **EP 2 401 354 B1**
(45) Date of publication and mention of the grant of the patent: **20.02.2013**
(21) Application number: 10712485.1
(22) Date of filing: 24.02.2010
(51) Int. Cl.: C12M 1/34, G01N 21/51

(54) **APPARATUS TO ANALYZE A BIOLOGICAL SAMPLE**
VORRICHTUNG ZUR ANALYSE EINER BIOLOGISCHEN PROBE
APPAREIL DESTINÉ À ANALYSER UN ÉCHANTILLON BIOLOGIQUE

(30) Priority: 25.02.2009 IT UD20090047
(43) Date of publication of application: 04.01.2012
(73) Proprietor: Alifax Holding S.p.A., 35020 Polverara (PD) (IT)
(72) Inventor: GALIANO, Paolo, I-35100 Padova (IT); MANSUTTI, Alessandro, I-33013 Gemona del Friuli (IT)
(74) Representative: Petraz, Gilberto Luigi
(86) International application number: PCT/IB2010/000369
(87) International publication number: WO 2010/097685

(56) References cited:
- WO-A-2006/021519
- JP-A- 58 173 455
- US-A- 3 773 426
- US-A- 5 012 119

## Description

### FIELD OF THE INVENTION

The present invention concerns an apparatus to analyze a liquid or solid biological sample, in particular to measure the bacterial cultural growth in a liquid culture ground, with the purpose of making a bacteriological and diagnostic search in general, or to measure turbidity for the purposes of a McFarland standardization for a sensitivity test, so as to make an analysis for sensitivity to antibiotics, or antibiogram, of a clinical or normal type.

The biological sample to be analyzed can be for example urine, bronchial aspirate, blood, diluted or lysed blood or other.

### BACKGROUND OF THE INVENTION

An analysis apparatus is known from document WO-A-2006/021519 in the name of the present Applicant, to detect the presence and possibly identify the bacteria in a biological sample, such as blood or urine.

In the known apparatus, based on light-scattering, a laser light emitter is provided which strikes a laser beam on a test tube or flacon made of transparent glass or plastic containing a culture liquid inoculated with the biological sample. The light diffused by the bacteria in suspension during growth is detected by suitable sensors which transmit the relative signals to a processing unit which determines the presence of bacteria, and possibly classifies or identifies the bacteria possibly present.

In this type of apparatus, which has shown itself to be efficacious and advantageous, the need has emerged to make everything more compact and safe for the worker and for the work environment. This because the known apparatus, due to the use of analysis recipients or flacons, entails a big overall bulk.

This also leads to a high production of potentially infected material, which represents a risk for the worker and requires a complex disposal process?.

Furthermore, given the ever greater number of analyses required, the problem of productivity of such analysis apparatuses is a very urgent one.

Purpose of the present invention is to achieve an analysis apparatus that allows to reduce the operating bulk and the production of potentially infected material.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purpose, an analysis apparatus according to the present invention is used to analyze a biological sample contained in a container, in particular but not only in order to perform a bacteriological search to measure the bacterial growth in order to verify the presence of bacteria, and possibly to classify and identify them.

The analysis apparatus according to the present invention comprises an examining device able to perform an optical measurement on the biological sample, based on light-scattering technology, at least of the bacterial growth in the biological sample. The examining device is provided with emitter means able to emit a first light beam toward the container of the biological sample and sensor means able to detect at least a light beam diffused from the container of the biological sample and to transmit a relative signal, correlated to said light beam diffused, to a control unit which is able to process said signal, directly or indirectly, so as to verify at least the bacterial growth, if any, in the biological sample.

According to the present invention, the container is disposed along a determinate lying plane and comprises at least a containing micro-element able to contain at least a part of the biological sample and inside which a liquid culture ground is provided so as to allow bacterial growth in the biological sample.

By containing micro-element, here and hereafter in the description, we mean a well or micro-well of a plate or micro-plate commonly used in microbiology, a reading cell or micro-cell, also known as cuvette or micro-cuvette, or in any case a container with very limited sizes with respect to the flacons or test tubes that are traditionally used, for example between 3 mm and 20 mm in height and between 3 mm and 20 mm in nominal diameter.

According to the present invention, the emitter means and sensor means can be located on each occasion in correspondence with the containing micro-element.

The sensor means comprise first sensor means disposed on the same side of the emitter means with respect to said lying plane and second sensor means disposed on the opposite side to the emitter means, always with respect to the lying plane of the container.

The first sensor means are able to detect a back-scattering radiation coming from the determinate containing micro-element analyzed on each occasion, while the second sensor means are able to detect a forward-scattering radiation, always coming from the containing micro-element.

According to the present invention, from said back-scattering and forward-scattering radiations respective first and second signals are derived, transmitted to the control unit. The signals are used to verify the bacterial growth and all the necessary analyses. According to one embodiment, the emitter means are located so that the first light beam strikes the determinate containing micro-element from above or below.

According to a variant, the emitter means are located vertical with respect to the lying plane of the container, so that the relative first light beam strikes the containing micro-element substantially perpendicularly.

Advantageously, the particular combination of the innovative characteristics of the present invention allows an accurate analysis of the sample using light-scattering technology, irrespective of the sizes or shape of the container of the sample, because the emitter means and sensors operate using a ray of light that is directed vertically.

According to another variant, the emitter means are located inclined with respect to the lying plane of the container so that the relative first light beam strikes the containing micro-element with a desired inclination by an angle other than 90° with respect to a vertical reference direction.

According to an advantageous embodiment, the container is a micro-plate of the type commonly used in microbiology (for example 96 or 384 wells) that develops along the lying plane, and said containing micro-elements are relative wells disposed in rows and columns along the lying plane.

Typically each of the micro-elements or wells has an upper aperture for the passage of the first light beam and the back-scattering radiation, and a bottom wall made of suitable material to allow the passage of the forward-scattering radiation.

Advantageously each of the wells has a lateral wall which is obscured to prevent the lateral diffusion of the light so as not to interfere with the other adjacent wells.

According to one embodiment the plates or micro-plates are made of polystyrene or methacrylate.

Advantageously, the first sensors and the second sensors are positioned, with respect to a determinate vertical direction, each at a defined angle comprised between about -90° and +90°.

It comes within the field of the present invention to use an examining device based on light-scattering technology to detect a back-scattering radiation coming from one side of a containing micro-element containing a biological sample in suspension in a liquid culture ground and a forward-scattering radiation from an opposite side of said containing micro-element, to measure at least the bacterial growth of the biological sample contained in the containing micro-element.

Advantageously, by said examining device of the light-scattering type, the growth curves of the bacteria possibly present in the sample analyzed are detected on one or more wells of the plate.

This detection, depending on the step of the analysis when it is made, can be used to determine the presence of bacteria (quick culture test), to identify the bacteria, or for the Raa test (residual antibiotic activity), for the clinical antibiogram test or other tests and analyses that require this type of detection.

If the present invention is applied to analyze lysed blood, the apparatus operates with light beams perpendicular to the transparent micro-well, to prevent possible interference from the lysed globules.

On the contrary, in applications to transparent liquids, where the optical travel by parallel or vertical reading assumes greater accuracy, it is advantageous to use micro-wells or micro-cuvettes with an obscured lateral wall.

One advantage of using micro-plates or other containing micro-elements combined with light-scattering detection, compared with containers of the type normally used for light-scattering, is that the micro-plate can be suitably filled with the volumes of samples necessary, including reagents and various liquids for the detection, including fluorescent components, preventing any waste of material.

Furthermore, by using micro-plates or other similar containers of limited size, such as micro-arrays, micro-cuvettes or other according to the present invention, the bulk of the analysis apparatus and the production of potentially infected material is reduced.

Moreover, the productivity of a single analysis apparatus of the type described above is considerably increased, as it can operate in quick succession on a plurality of adjacent containing micro-elements, each with a precise and sufficient, not excessive, quantity of sample and culture liquid, thus limiting wastes of material and analysis time.

The present invention also comprises a method to analyze a biological sample contained in a container disposed along a determinate lying plane and comprising at least a containing micro-element able to contain at least part of the biological sample and inside which a liquid culture ground is provided to allow bacterial growth in the biological sample. The method provides an examination step in which an optical measurement is carried out, based on light-scattering technology, at least of the bacterial growth in the biological sample, by emitting a first light beam toward the biological sample, detecting at least a light beam diffused from the container of the biological sample and by processing, directly or indirectly, signals correlated to said light beam diffused. According to the present invention, the method provides to detect, on the same side with respect to the lying plane associated with the containing micro-element from which said first light beam is emitted, a back-scattering radiation coming from the determinate containing micro-element and, on the side opposite the lying plane associated with the containing micro-element, a forward-scattering radiation coming from the determinate containing micro-element. The method also provides to derive said signals from the back-scattering and forward-scattering radiations.

### BRIEF DESCRIPTION OF THE DRAWINGS

These and other characteristics of the present invention will become apparent from the following description of a preferential form of embodiment, given as a non-restrictive example with reference to the attached drawings wherein:
- fig. 1 is a schematic representation of a container used with the present invention;
- fig. 2 is a schematic representation of part of the apparatus according to the present invention;
- fig. 3 is an enlarged detail of fig. 2;
- fig. 4 is a variant of a part of the apparatus in fig. 2;
- fig. 5 is a schematic representation of an embodiment of the apparatus according to the present invention.

### DETAILED DESCRIPTION OF A PREFERENTIAL FORM OF EMBODIMENT

With reference to the attached drawings, an apparatus 10 according to the invention is used to analyze a biological sample 11, in particular to measure the bacterial growth and to identify the presence of bacteria contained in the biological sample. The apparatus 10 applies the light-scattering technique to small size containers, such as for example micro-plates (96 wells) or micro-arrays (384 wells), micro-cuvettes or glass micro-cells, micro-cuvettes with the lateral wall obscured (to prevent the diffusion of the light beam).

The same apparatus 10 can be used to measure turbidity for the purposes of McFarland standardization for a sensitivity test, to carry out an analysis of sensitivity to antibiotics, or antibiogram, of the clinical or normal type.

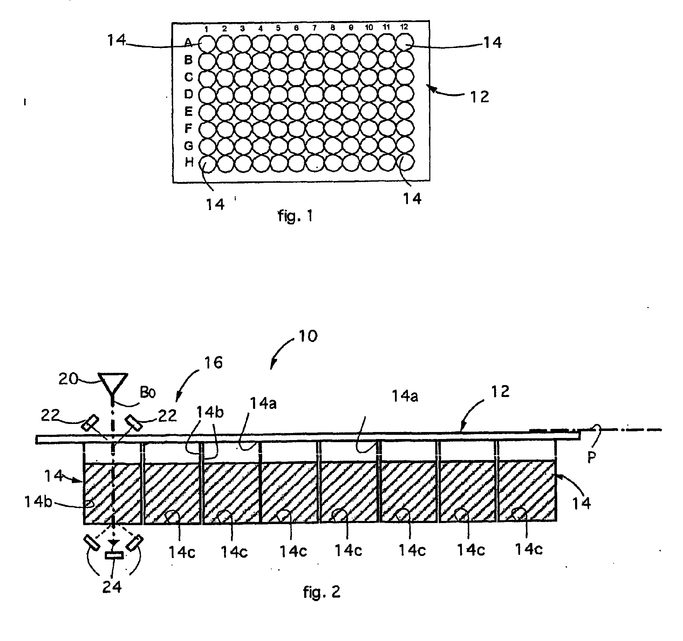

In this case, the apparatus 10 comprises a micro-plate 12, formed by 96 containing micro-elements or wells 14 (fig. 1), disposed along a substantially horizontal lying plane P (fig. 2).

The wells 14 function as containers for bacterial growth in a suitable liquid culture ground, for example eugenic medium, and for the possible biochemical reactions used to recognize the presence and/or identify the type of bacteria in the biological sample 11 to be analyzed.

The plates 12 can be suitably thermostated, for example at a temperature comprised between about 35°C and 37°C, to promote the bacterial growth, and preferentially subjected to stirring.

For use with light-scattering technology, as required by the present invention, the plates 12 and the wells 14 are compatible with the desired electromagnetic radiation, preferably transparent or permeable to visible light.

The plates 12 employed, therefore, preferably have good optical qualities, typically with great clarity and uniformity, so that they have a high capacity of letting the light pass through the wells 14 in the analysis. For example, the plates 12 are made of polystyrene or methacrylate, which embody the above optimum optical properties.

Each well 14 has an upper aperture 14a, for the sample to be inserted and for the light to pass, and a bottom wall 14c, also permeable to light.

In the specific solution, where there are adjacent wells 14 in large quantities, their lateral walls 14b are blackened, or in any case rendered not transparent to light, so as to eliminate possible optical interferences of one well 14 with the adjacent wells. Typically, before inoculation, the biological sample is preserved in a test tube and a part of it is picked up, advantageously by means of a movement and selection unit, not shown in the drawings, and deposited in one or more of the wells 14 of the plate 12, to perform the desired analyses.

The apparatus 10 also comprises an examining device 16 based on light-scattering technology, by means of which a step of detection is carried out on one or more of the wells 14, in which suitable signals are generated by means of which the bacterial growth or inhibition kinetics in each well 14 are assessed, as illustrated hereafter in the description (figs. 2, 3 and 5).

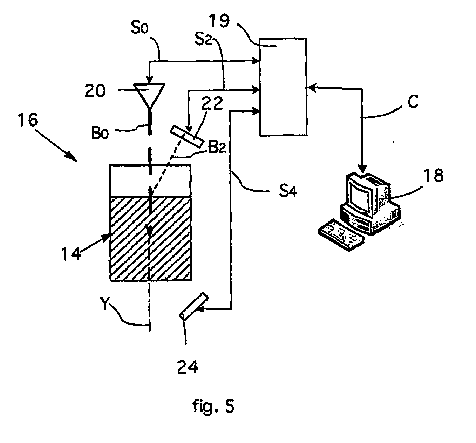

The apparatus 10 also comprises a control unit 18, for example an electronic calculator, in whose memory a computer program is loaded to manage the examining device 16. The control unit 18 is shown for convenience only in fig. 5. The control unit 18 is associated, by means of a hardware connection C, to an interface card 19 which is able to control the activation/de-activation of the examining device 16 (fig. 5), as shown schematically by the command and control signal S0.

The data collected by the examining device 16 are sent to the control unit 18 by the interface card 19, which amplifies, filters and processes the data collected.

In this way, the control unit 18 is able to evaluate the bacterial growth or inhibition kinetics in each well 14 (bacterial growth test).

The control unit 18 can also activate the sample movement and selection unit, if there is one.

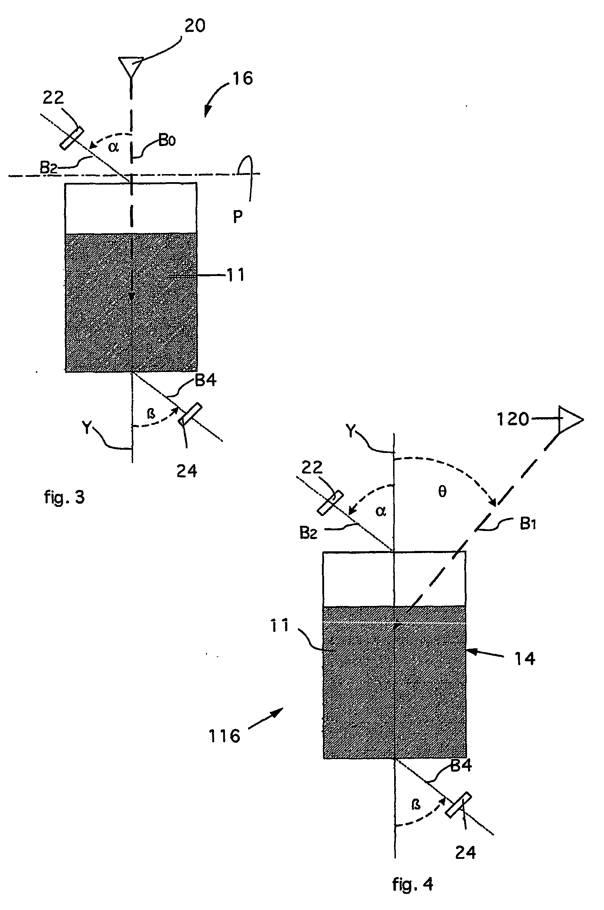

According to the embodiment shown in figs. 2 and 3, the examining device 16 comprises a light emitter 20, disposed vertically above or below the micro-plate 12 with respect to said plane P, in correspondence with the various wells 14 which make up the micro-plate 12; the emitter 20 is able to emit a laser light beam, polarized and collimated, indicated by B0, toward the well 14 to be analyzed, with an inclination of 90° with respect to the lying plane P of the micro-plate 12, which is an advantageous solution in the analysis of lysed blood so as to prevent possible interferences of the lysed globules. The light beam B0 is diffused from the relative well 14 of the micro-plate 12, in particular by the biological sample growing in the liquid culture ground inside the well 14 of the micro-plate 12.

A plurality of sensors 22, 24 are associated with the emitter 20, and are located vertically above and below the lying plane P of the micro-plate 12.

In particular, according to the embodiment shown in fig. 2, two first sensors 22 are provided, located on the same side of the emitter 20 with respect to the micro-plate 12, and three second sensors 24, located on the opposite side of the emitter with respect to the lying plane P of the micro-plate 12. It is understood that the number of sensors can be different from this.

Both the first 22 and the second 24 sensors are positioned at different angles with respect to the vertical direction, indicated by Y and coincident with the path of the light beam B0. The angles are indicated by α and β in fig. 3 and are typically comprised between -90° and +90° with respect to the vertical Y. In the solution given as an example in fig. 2, there are two upper sensors 22 disposed respectively at about +45° and -45° and three lower sensors, of which two external disposed respectively at about +45° and -45°, and a central one disposed at 0°, that is, aligned with the vertical Y.

In a different form of embodiment, not shown, the first and second sensors 22, 24 can be positioned at different angles, for example at about 0°, 30° and 90°.

Furthermore, the sensors 22, 24 are positioned at a variable distance from the relative well 14, according to the requirements of the analysis. In fact, once struck by the light beam B0 emitted, the biological sample 11 in the well 14, with the presence of duplicating bacteria, emits beams of diffused light B2, B4 which the control unit 18 processes so as to supply specific curves that express the development of the bacterial growth over time.

The sensors 22 and 24 periodically detect the beams B2, B4 of diffused light emitted by the biological sample 11 contained in the well 14 and transmit them as signals S2, S4 to the control unit 18.

In particular, the first sensors 22 periodically detect the back-scattering, indicated by the light beam B2, of the light striking the well 14, whereas the second sensors 24 detect the forward-scattering, indicated by the light beam B4, of the light through the well 14.

Fig. 3 is a detail referring to a single well 14, in which a single first sensor 22 and a single second sensor 24 are used, disposed on opposite sides with respect to the lying plane P of the micro-plate 12.

The sensors 22 and 24 transmit the relative signals S2, S4, correlated to the light beams B2, B4 detected, to the interface card 19, which converts them from analog to digital, transmitting the relative value of forward-scattering and back-scattering to the control unit 18 which processes the signals for the required analyses and evaluations.

Once converted, the signals S2, S4 allow to determine a first and a second curve, respectively associated with each of the sensors 22, 24, of the development over time of the turbidity of the bacterial suspension, that is, of the bacterial growth. The control unit 18 processes the signals S2, S4 so as to determine, from said curves, two corresponding differential curves.

Each differential curve is given by the difference respectively between the first curve and a first instantaneous value of turbidity obtained at the start of the detection, detected by the first sensor 22, and between the second curve and a second instantaneous value of turbidity at the start of the detection, detected in correspondence with the second sensor 24.

The control unit 18 advantageously comprises memorization means in which classification data are memorized, by means of which, from the development of the two differential curves, the type of bacterium present in the biological sample where the bacterial growth takes place, or the family it belongs to, is found.

The first curve derived from the signal obtained by the first sensor 22 relates to the presence of bacteria and consequent measurement of the bacterial load over time. The second curve derived from the signal obtained by the second sensor 24 on the contrary is more characterized by the morphology of the bacteria.

Starting from the differential curves, specific mathematical parameters are extrapolated for each curve, based on non-linear regression models. It is also possible to combine the homologous parameters of the two curves (for example by calculating ratios, or differences or sums) so as to obtain further derived parameters.

The totality of these parameters provides a synthetic description of the characteristics of the curves of the bacterium present in the sample examined.

Fig. 4 shows a variant embodiment of the examining device 16, indicated for convenience by the reference number 116, where parts identical to those shown in the embodiment in figs. 1-3 are identified by the same reference numbers. The examining device 116 provides an emitter 120 that is not located perpendicular with respect to the micro-plate but is inclined by a determinate angle θ with respect to the vertical Y. The positions of the sensors 22 and 24 remain as described in the various possibilities in figs. 1-3. In this way, at least part of the light-scattering components generated by a final segment of the volume of illuminated sample, and directed at 90° with respect to the direction of the radiation emitted B1, passes through the bottom wall 14c of the well 14 to be detected by the sensors 24, instead of being absorbed by the lateral walls 14b, as in the case of an emitter 120 located perpendicular with respect to the micro-plate.

## Claims

1. Apparatus to analyze a biological sample (11) contained in a container (12), comprising an examining device (16) able to perform an optical measurement on the biological sample (11), based on light-scattering technology, at least of the bacterial growth in the biological sample (11) and provided with emitter means (20) able to emit a first light beam (B0) toward the container (12) of the biological sample (11) and sensor means (22, 24) able to detect at least a light beam diffused from the container (12) of the biological sample and to transmit a relative signal (S2, S4), correlated to said light beam diffused, to a control unit (18) which is able to process, directly or indirectly, said signal (S2, S4), in order to verify at least the possible bacterial growth in the biological sample (11), **characterized in that** said container (12) is disposed along a determinate lying plane (P) and comprises at least a containing micro-element (14) able to contain at least a part of the biological sample (11) and inside which a liquid culture ground is provided so as to allow bacterial growth in the biological sample (11), said emitter means (20) and sensor means (22, 24) being able to be located on each occasion in correspondence with the containing micro-element (14), said sensor means (22, 24) comprising first sensor means (22) disposed on the same side of the emitter means (20) with respect to said lying plane (P), and second sensor means (24) disposed on the opposite side of the emitter means (20) with respect to said lying plane (P), said first sensor means (22) being able to detect a back-scattering radiation (B2) coming from the determinate containing micro-element (14) and said second sensor means (24) being able to detect a forward-scattering radiation (B4) coming from the determinate containing micro-element (14), from which back-scattering radiation (B2) and forward-scattering radiation (B4) derive respective first (S2) and second (S4) signals transmitted to the control unit (18).

2. Apparatus as in claim 1, **characterized in that** said emitter means (20) are placed so that the first light beam (B0) strikes, from above or from below, on the determinate containing micro-element (14).

3. Apparatus as in claim 1 or 2, **characterized in that** said emitter means (20) are placed vertically with respect to the lying plane (P) of said container (12), so that the relative first light beam (B0) strikes substantially perpendicularly on said containing micro-element (14).

4. Apparatus as in claim 1 or 2, **characterized in that** said emitter means (20) are placed inclined with respect to the lying plane (P) of said container (12) so that the relative first light beam (B0) strikes on said containing micro-element (14) with a desired inclination of an angle (θ) different from 90° with respect to a vertical direction (Y).

5. Apparatus as in any claim hereinbefore, **characterized in that** said container is a micro-plate (12) which develops along said lying plane (P) and said micro-containing elements are relative wells (14) disposed in rows and columns along said lying plane (P).

6. Apparatus as in claim 5, **characterized in that** each of the wells (14) has an upper opening (14a) for the passage of the first light beam (B0) and of the back-scattering radiation (B2) and a bottom wall (14c) made of material suitable to allow the passage of the forward-scattering radiation (B4).

7. Apparatus as in claim 5 or 6, **characterized in that** each of the wells (14) has a lateral wall (14b), which is obscured so as to impede the lateral diffusion of light in order not to interfere with the other adjacent wells (14).

8. Apparatus as in any claim hereinbefore, **characterized in that** the plates or micro-plates (12) are made of polystyrene or methacrylate.

9. Apparatus as in any claim hereinbefore, **characterized in that** the first sensors means (22) and the second sensors means (24) are positioned, with respect to a determined vertical direction (Y), each at a defined angle (a, β) comprised between about -90° and + 90°.

10. Method to analyze a biological sample (11) contained in a container (12), disposed along a determinate lying plane (P) and comprising at least a containing micro-element (14) able to contain at least a part of the biological sample (11) and inside which a liquid culture ground is provided so as to allow bacterial growth in the biological sample (11), said method providing an examination step in which an optical measurement is made, based on light-scattering technology, at least of the bacterial growth in the biological sample (11) by emitting a first light beam (B0) toward the container (12) of the biological sample (11), by detecting at least a light beam diffused from the container (12) of the biological sample (11) and by processing, directly or indirectly, signals (S2, S4) correlated to said light beam diffused, **characterized in that** it provides to detect, on the same side with respect to the lying plane (P) associated with the containing micro-element (14) from which said first light beam (B0) is emitted, a back-scattering radiation (B2) coming from the determinate containing micro-element (14) and, from the opposite side with respect to the lying plane (P) associated with the containing micro-element (14), a forward-scattering radiation (B4) coming from the determinate containing micro-element (14), deriving said signals (S2, S4) from said back-scattering radiations (B2) and forward-scattering radiations (B4).

## Patentansprüche

1. Einrichtung zum Analysieren einer biologischen Probe (11), welche in einem Behältnis (12) enthalten ist, welche eine Untersuchungsvorrichtung (16) enthält, welche dazu ausgelegt ist, eine optische Messung der biologischen Probe (11), basierend auf einer Lichtzerstreuungs-Technologie, von zumindest dem bakteriellen Wachstum in der biologischen Probe (11) durchzuführen, und welche mit Emitter-Elementen (20), welche dazu ausgelegt sind, einen ersten Lichtstrahl (B0) in Richtung des Behältnisses (12) der biologischen Probe (11) zu emittieren, und Sensor-Elementen (22, 24) bereitgestellt ist, welche dazu ausgelegt sind, zumindest einen Lichtstrahl, welcher vom Behältnis (12) der biologischen Probe diffundiert ist, zu erfassen, und ein Relativsignal (S2, S4), welches mit dem diffundierten Lichtstrahl korreliert ist, an eine Steuereinheit (18) zu übertragen, welche dazu ausgelegt ist, direkt oder indirekt, das Signal (S2, S4) zu verarbeiten, um zumindest das mögliche bakterielle Wachstum in der biologischen Probe (11) zu verifizieren, **dadurch gekennzeichnet, dass** das Behältnis (12) entlang einer festgelegten liegenden Ebene (P) angeordnet ist und zumindest ein umfasstes Mikroelement (14) enthält, welches dazu ausgelegt ist, zumindest einen Teil der biologischen Probe (11) zu umfassen, und innerhalb dessen eine flüssige Kulturmasse bereitgestellt ist, um ein bakterielles Wachstum in der biologischen Probe (11) zu erlauben, wobei die Emitter-Elemente (20) und Sensor-Elemente (22, 24) dazu ausgelegt sind, bei jedem Anlass in Übereinstimmung mit dem umfassten Mikroelement (14) positioniert zu werden, wobei die Sensor-Elemente (22, 24) ein erstes Sensor-Element (22), welches auf der gleichen Seite des Emitter-Elements (20) mit Bezug auf die liegende Ebene (P) angeordnet ist, und ein zweites Sensor-Element (24), welches auf der gegenüberliegenden Seite des Emitter-Elements (20) mit Bezug auf die liegende Ebene (P) angeordnet ist, enthalten, wobei das erste Sensor-Element (22) dazu ausgelegt ist, eine rückwärts gerichtete zerstreute Strahlung (B2) zu erfassen, welche von dem festgelegten umfassten Mikroelement (14) stammt, und das zweite Sensor-Element (24) dazu ausgelegt ist, eine vorwärts gerichtete zerstreute Strahlung (B4) zu erfassen, welche von dem festgelegten umfassten Mikroelement (14) stammt, wobei von der rückwärts gerichteten zerstreuten Strahlung (B2) und der vorwärts gerichteten zerstreuten Strahlung (B4) ein jeweiliges erstes (S2) und zweites (S4) Signal hergeleitet werden, welche an die Steuereinheit (18) übertragen werden.

2. Einrichtung nach Anspruch 1, **dadurch gekennzeichnet, dass** die Emitter-Elemente (20) derart positioniert sind, dass der erste Lichtstrahl (B0) von oberhalb oder von unterhalb auf das festgelegte umfasste Mikroelement (14) auftrifft.

3. Einrichtung nach Anspruch 1 oder 2, **dadurch gekennzeichnet, dass** die Emitter-Elemente (20) mit Bezug auf die liegende Ebene (P) des Behältnisses (12) vertikal positioniert sind, so dass der relative erste Lichtstrahl (B0) im Wesentlichen senkrecht auf das umfasste Mikroelement (14) auftrifft.

4. Einrichtung nach Anspruch 1 oder 2, **dadurch gekennzeichnet, dass** die Emitter-Elemente (20) mit Bezug auf die liegende Ebene (P) des Behältnisses (12) geneigt positioniert sind, so dass der relative erste Lichtstrahl (B0) mit einer gewünschten Winkelneigung (Θ) auf das umfasste Mikroelement (14) auftrifft, welche sich mit Bezug auf eine vertikale Richtung (Y) um 90° unterscheidet.

5. Einrichtung nach einem der vorhergehenden Ansprüche, **dadurch gekennzeichnet, dass** das Behältnis eine Mikroplatte (12) ist, welche sich entlang der liegenden Ebene (P) erstreckt, und die umfassten Mikroelemente relative Quellen (14) sind, welche entlang der liegenden Ebene (P) in Reihen und Spalten angeordnet sind.

6. Einrichtung nach Anspruch 5, **dadurch gekennzeichnet, dass** jede der Quellen (14) eine obere Öffnung (14a) für den Durchgang des ersten Lichtstrahls (B0) und der rückwärts gerichteten zerstreuten Strahlung (B2), und eine untere Wand (14c) hat, welche aus einem Material erstellt ist, welches dazu ausgelegt ist, den Durchgang der vorwärts gerichteten zerstreuten Strahlung (B4) zu ermöglichen.

7. Einrichtung nach Anspruch 5 oder 6, **dadurch gekennzeichnet, dass** jede der Quellen (14) eine Seitenwand (14b) hat, welche derart verschleiert ist, dass die seitliche Lichtdiffusion verhindert wird, um nicht mit den weiteren angrenzenden Quellen (14) zu interferieren.

8. Einrichtung nach einem der vorhergehenden Ansprüche, **dadurch gekennzeichnet, dass** die Platten oder Mikroplatten (12) aus Polystyren oder Methacrylat erstellt sind.

9. Einrichtung nach einem der vorhergehenden Ansprüche, **dadurch gekennzeichnet, dass** die ersten Sensor-Elemente (22) und die zweiten Sensor-Elemente (24) mit Bezug auf eine bestimmte Vertikalrichtung (Y) positioniert sind, jeweils bei einem bestimmten Winkel (α, β), welcher in einem Bereich zwischen etwa -90° und +90° liegt.

10. Verfahren zum Analysieren einer biologischen Probe (11), welche in einem Behältnis (12) umfasst ist, welches entlang einer festgelegten liegenden Ebene (P) angeordnet ist und zumindest ein umfasstes Mikroelement (14) enthält, welches dazu ausgelegt ist, zumindest einen Teil der biologischen Probe (11) zu umfassen, und innerhalb dessen eine flüssige Kulturmasse bereitgestellt ist, um ein bakterielles Wachstum in der biologischen Probe (11) zu ermöglichen, wobei das Verfahren einen Untersuchungsschritt bereitstellt, bei welchem eine optische Messung basierend auf einer Lichtzerstreuungs-Technologie über zumindest das bakterielle Wachstum in der biologischen Probe (11) vorgenommen wird, indem ein erster Lichtstrahl (B0) in Richtung des Behältnisses (12) der biologischen Probe (11) emittiert wird, indem zumindest ein Lichtstrahl erfasst wird, welcher vom Behältnis (12) der biologischen Probe (11) diffundiert wird, und indem, direkt oder indirekt, Signale (S2, S4) verarbeitet werden, welche mit dem diffundierten Lichtstrahl korreliert sind, **dadurch gekennzeichnet, dass** es ein Erfassen, auf der gleichen Seite mit Bezug auf die liegende Ebene (P), in Zusammenhang mit dem umfassten Mikroelement (14), von welchem der erste Lichtstrahl (B0) emittiert wird, von einer rückwärts gerichteten zerstreuten Strahlung (B2), welche von dem festgelegten umfassten Mikroelement (14) stammt, und, von der gegenüberliegenden Seite mit Bezug auf die liegende Ebene (P), in Zusammenhang mit dem umfassten Mikroelement (14), von einer vorwärts gerichteten zerstreuten Strahlung (B4), welche von dem festgelegten umfassten Mikroelement (14) stammt, enthält, wobei die Signale (S2, S4) von den rückwärts gerichteten zerstreuten Strahlungen (B2) und den vorwärts gerichteten zerstreuten Strahlungen (B4) hergeleitet werden.

## Revendications

1. Appareil destiné à analyser un échantillon biologique (11) contenu dans un conteneur (12), comprenant un dispositif d'examen (16) apte à effectuer une mesure optique de l'échantillon biologique (11), basée sur la technique de diffusion de la lumière, au moins de la croissance bactérienne dans l'échantillon biologique (11) et doté d'un moyen d'émission (20) apte à émettre un premier faisceau lumineux (B0) en direction du conteneur (12) de l'échantillon biologique (11) et de moyens de détection (22, 24) aptes à détecter au moins un faisceau lumineux diffusé depuis le conteneur (12) de l'échantillon biologique et à transmettre un signal correspondant (S2, S4), corrélé audit faisceau lumineux diffusé, à une unité de contrôle (18) capable de traiter, directement ou indirectement, ledit signal (S2, S4) afin de vérifier au moins l'éventuelle croissance bactérienne dans l'échantillon biologique (11), **caractérisé en ce que** ledit conteneur (12) est placé le long d'un plan horizontal (P) déterminé et comprend au moins un micro-élément conteneur (14) pouvant contenir au moins une partie de l'échantillon biologique (11), et dans lequel est prévu un milieu liquide de culture afin de permettre la croissance bactérienne dans l'échantillon biologique (11), lesdits moyens d'émission (20) et de détection (22, 24) pouvant se trouver à chaque occasion en correspondance avec le micro-élément conteneur (14), lesdits moyens de détection (22, 24) comprenant des premiers moyens de détection (22) placés du même côté que le moyen d'émission (20) par rapport audit plan horizontal (P), et des seconds moyens de détection (24) placés du côté opposé au moyen d'émission (20) par rapport audit plan horizontal (P), lesdits premiers moyens de détection (22) étant aptes à détecter un rayonnement de rétrodiffusion (B2) provenant du micro-élément conteneur (14) déterminé, et lesdits seconds moyens de détection (24) étant aptes à détecter un rayonnement de prodiffusion (B4) provenant du micro-élément conteneur (14) déterminé, les premier (S2) et second (S4) signaux transmis à l'unité de contrôle (18) étant respectivement tirés du rayonnement de rétrodiffusion (B2) et du rayonnement de prodiffusion (B4),

2. Appareil selon la revendication 1, **caractérisé en ce que** ledit moyen d'émission (20) est placé de sorte que le premier faisceau lumineux (B0) touche, par dessus ou par dessous, le micro-élément conteneur (14) déterminé.

3. Appareil selon la revendication 1 ou 2, **caractérisé en ce que** ledit moyen d'émission (20) est placé verticalement par rapport au plan horizontal (P) dudit conteneur (12), de sorte que le premier faisceau lumineux (B0) correspondant touche ledit micro-élément conteneur (14) sensiblement à la perpendiculaire.

4. Appareil selon la revendication 1 ou 2, **caractérisé en ce que** ledit moyen d'émission (20) est incliné par rapport au plan horizontal (P) dudit conteneur (12), de sorte que le premier faisceau lumineux (B0) correspondant touche ledit micro-élément conteneur (14) suivant l'inclinaison souhaitée d'angle (θ), différent de 90° par rapport à la verticale (Y).

5. Appareil selon l'une quelconque des revendications précédentes, **caractérisé en ce que** ledit conteneur est une microplaque (12) disposée dans ledit plan horizontal (P), et **en ce que** lesdits micro-éléments conteneurs sont des puits (14) correspondants disposés en rangées et en colonnes le long dudit plan horizontal (P).

6. Appareil selon la revendication 5, **caractérisé en ce que** chacun des puits (14) possède une ouverture supérieure (14a) pour le passage du premier faisceau lumineux (B0) et du rayonnement de rétrodiffusion (B2), et une paroi de fond (14c) constituée d'un matériau apte à permettre le passage du rayonnement de prodiffusion (B4).

7. Appareil selon la revendication 5 ou 6, **caractérisé en ce que** chacun des puits (14) possède une paroi latérale (14) qui est assombrie de manière à bloquer la diffusion latérale de lumière et à éviter ainsi d'interférer avec les autres puits adjacents (14).

8. Appareil selon l'une quelconque des revendications précédentes, **caractérisé en ce que** les plaques ou microplaques (12) sont en polystyrène ou en méthacrylate.

9. Appareil selon l'une quelconque des revendications précédentes, **caractérisé en ce que** chacun des premiers moyens de détection (22) et des seconds moyens de détection (24) est positionné, par rapport à une verticale (Y) déterminée, à un angle défini (α, β) approximativement compris entre - 90° et + 90°.

10. Procédé destiné à analyser un échantillon biologique (11) contenu dans un conteneur (12) placé le long d'un plan horizontal (P) déterminé et comprenant au moins un micro-élément conteneur (14) pouvant contenir au moins une partie de l'échantillon biologique (11), et dans lequel est prévu un milieu liquide de culture afin de permettre la croissance bactérienne dans l'échantillon biologique (11), ledit procédé prévoyant une étape d'examen au cours de laquelle est effectuée une mesure optique, basée sur la technique de diffusion de la lumière, au moins de la croissance bactérienne dans l'échantillon biologique (11) en émettant un premier faisceau lumineux (B0) en direction du conteneur (12) de l'échantillon biologique (11), en détectant au moins un faisceau lumineux diffusé par le conteneur (12) de l'échantillon biologique (11) et en traitant, directement ou indirectement, les signaux (S2, S4) corrélés audit faisceau lumineux diffusé, **caractérisé en ce qu'**il prévoit de détecter, du même côté par rapport au plan horizontal (P) associé au micro-élément conteneur (14) à partir duquel est émis ledit premier faisceau lumineux B0), un rayonnement de rétrodiffusion (B2) provenant du micro-élément conteneur (14) déterminé et, du côté opposé par rapport au plan horizontal (P) associé au micro-élément conteneur (14), un rayonnement de prodiffusion (B4) provenant du micro-élément conteneur (14) déterminé, lesdits signaux (S2, S4) étant tirés desdits rayonnements de rétrodiffusion (B2) et de prodiffusion (B4).
